(19) Europäisches Patentamt
European Patent Office
Office européen des brevets

(11) Publication number: **0 132 540**
**A1**

(12) **EUROPEAN PATENT APPLICATION**

(21) Application number: 84106186.4

(22) Date of filing: 22.06.82

(51) Int. Cl.⁴: A 61 K 31/19
A 61 K 31/235, A 61 K 31/535

(30) Priority: 06.05.82 JP 75782/82
22.06.81 JP 96376/81

(43) Date of publication of application:
13.02.85 Bulletin 85/7

(84) Designated Contracting States:
BE IT

(60) Publication number of the earlier application in accordance with Art. 76 EPC: 0 068 408

(71) Applicant: Mochida Pharmaceutical Co., Ltd.
1-7, Yotsuya Shinjuku-ku
Tokyo(JP)

(72) Inventor: Mochida, Ei
2-5-4, Komagome Toshima-ku
Tokyo(JP)

(72) Inventor: Suzuki, Yasuo
234-29, Oaza Sashima
Kawaguchi-shi Saitama-ken(JP)

(72) Inventor: Yamaguchi, Kazuo
4-18-7, Higashi-cho
Koganei-shi Tokyo(JP)

(72) Inventor: Ohnishi, Haruo
6-4-14, Higashi Funubashi
Funabashi-shi Chiba-ken(JP)

(74) Representative: Hansen, Bernd, Dr.rer.nat. et al,
Hoffmann, Eitle & Partner Patentanwälte Arabellastrasse 4
D-8000 München 81(DE)

# (54) Antiviral compositions.

(57) The present invention relates to antiviral compositions which contain as an active component aminosulfonylhalogenobenzoic acid derivatives of the formula (I):

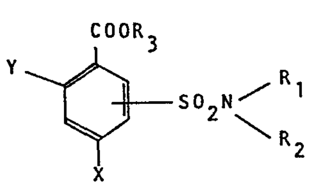

(I)

wherein X denotes a fluorine atom, a chlorine atom, a bromine atom, Y denotes a hydrogen atom or a chlorine atom, $R_1$ denotes a hydrogen atom or a methyl group, $R_2$ denotes a hydrogen atom, a methyl group, an ethylgroup, an isopropyl group or a phenyl group, or $R_1$ and $R_2$ together with the nitrogen atom in the formula may form a morpholino group, and $R_3$ denotes a hydrogen atom, a methyl group, and isopropyl group, a n-butyl group, or an allyl group, and the salts thereof.

EP 0 132 540 A1

Divisional application of
82 105 460.8

## Antiviral compositions

The present invention relates to antiviral compositions and, more particularly, to antiviral compositions which contain aminosulfonylhalogenobenzoic acid derivatives or salts thereof which are effective for therapeutic treatment of various infectious diseases which are caused from viruses, for example, upper respiratory infection, pneumonia, Bronchitis and so on.

Vaccines which have been used for viral diseases are slow-acting and their utility is limited only to preventive use. Moreover, since antigenicity of objective viruses often changes, the effect of vaccines have not been sufficient.

Under these circumstances, the present inventors have carried out intensive studies to find out pharmaceuticals which have fast-acting therapeutic effects.

As a result, the inventors found out that aminosulfonylhalogenobenzoic acid derivatives meet the above requirement, thus completed the present invention.

Accordingly, the object of the present invention is to provide pharmaceutical compositions having antiviral activity which contain aminosulfonylhalogenobenzoic acid derivatives which are effective for therapeutic treatment of various infectious diseases caused from viruses, such as upper respiratory infection, pneumonia, Bronchitis and so on.

Another object of the present invention is to provide a method of treating virus diseases by said compositions.

Further objects and advantages and features of the present invention will become apparent during the course of the description with follows.

The present invention relates to antiviral compositions which contain as an active component aminosulfonylhalogenobenzoic acid derivatives of the formula (I):

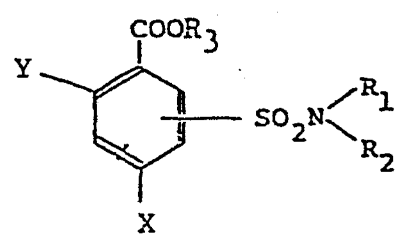

(I)

wherein X denotes a fluorine atom, a chlorine atom, a bromine atom, Y denotes a hydrogen atom or

a chlorine atom, $R_1$ denotes a hydrogen atom or a methyl group, $R_2$ denotes a hydrogen atom, a methyl group, an ethylgroup, an isopropyl group or a phenyl group, or $R_1$ and $R_2$ together with the nitrogen atom in the formula may form a morpholino group, and $R_3$ denotes a hydrogen atom, a methyl group, an isopropyl group, a n-butyl group, or an allyl group, and the salts thereof.

The compounds which are the main ingredients of antiviral compositions according to the present invention can be prepared as follows. Namely, halogenobenzoic acid is heated in chlorosulfonic acid to obtain chlorosulfonylhalogenobenzoic acid. (Brit. 8961,137) Chlorosulfonylhalogenobenzoic acid thus obtained is reacted with an amine of the formula (II) $HN{<}^{R_1}_{R_2}$ at room temperature in a solvent such as water, dioxane, tetrahydrofuran, chloroform, dichloromethane, benzen and so on or in a mixed solvent of water with one of these organic solvents, in the presence of deoxidizing agent, for example, inorganic base such as sodium hydroxide, potassium hydroxide, sodium carbonate, potassium carbonate, sodium bicarbonate and so on or an organic base such as triethyl amine, pyridine and so on, or using $HN{<}^{R_1}_{R_2}$ per se as deoxydizing agent.

Aminosulfonylhalogenobenzoic ester can be prepared as follows: Halogenoaminosulfonylbenzoic acid is heated in chlorothionyl to obtain halogenoaminosulfonyl benzoic chloride (GB-PS No. 915,259). Halogenoaminosulfonylbenzoic chloride obtained in this way is reacted with an alcohol of the following formula (III)

$$R_4OH \qquad (III)$$

wherein $R_4$ has the same meaning as $R_3$ excluding hydrogen atom. The reaction is performed at room temperature or under heating when necessary in a solvent such as dioxane, tetrahydrofuran, chloroform, dichloromethane, and benzene. Alternatively, the alcohol showing the above formula (III) per se is used as a solvent.

The physicochemical properties of the aminosulfonylhalogenobenzoic acid derivatives thus produced are shown in Table 1.

Table 1. Physicochemical Properties

(the term (dec) means "decomposition")

| Compound | Chemical Name | Melting Point (°C) | Property |
|---|---|---|---|
| 1 | 4-chloro-3-methylamino-sulfonylbenzoic acid | 236.5 - 239 | white crystalline powder; insoluble in benzene, hexane; soluble in hot water, methanol |
| 2 | 4-chloro-3-ethylamino-sulfonylbenzoic acid | 190.5 - 192.5 | white crystalline powder; insoluble in benzene, hexane; soluble in hot water, methanol |
| 3 | 4-chloro-3-dimethyl-aminosulfonylbenzoic acid | 248.5 - 249.5 | white crystalline powder; insoluble in benzene, hexane; soluble in hot water, methanol |
| 4 | 4-chloro-3-isopropyl-aminoslfonylbenzoic acid | 189 - 191 | white crystalline powder; insoluble in benzene, hexane; soluble in hot water, methanol |
| 5 | 3-anilinosulfonyl-4-chlorobenzoic acid | 208 - 210 | white crystalline powder; insoluble in benzene, hexane; soluble in hot water, methanol |

| Compound | Chemical Name | Melting Point (°C) | Property |
|---|---|---|---|
| 6 | 4-chloro-3-morpholino-sulfonylbenzoic acid | 187 - 189.5 | white crystalline powder; insoluble in benzene, hexane; soluble in hot water, methanol |
| 7 | 5-aminosulfonyl-2,4-dichlorobenzoic acid | 232 - 233.5 | white crystalline.powder; insoluble in benzene, hexane; soluble in hot water, methanol |
| 8 | 3-aminosulfonyl-4-fluoromethylbenzoate | 122 - 124 | white crystalline powder |
| 9 | 3-aminosulfonyl-4-bromo-isopropylbenzoate | 187 - 190 | white crystalline powder |
| 10 | 3-aminosulfonyl-4-bromo-n-butylbenzoate | 100 - 105 | white crystalline powder |
| 11 | 5-aminosulfonyl-2,4-dichloro-allylbenzoate | 109 -112 | white crystalline powder |

The aminosulfonylhalogenobenzoic acid derivatives thus produced can be converted to the pharmaceutically acceptable salts. The salt may be sodium salt, potassium salt, lithium salt, ammonium salt, calcium salt, barium salt and so on.

The following is the explanation of the effectiveness, safety, method for application and dosage of aminosulfonylhalogenobenzoic acid derivatives thus obtained.

Experimental Example 1   Antiviral Activity in Cultured Cells

Antiviral activity of compound of the invention was determined according to the method described by Marks. (Antimicrob, Agents Chemother., Volume 6, Pages 34 ~ 38, 1974).

Monolayer culture of MDCK, Vero and HEL cells were inoculated with 0.1 ml of serial 10-fold dilution of the virus in Eagle's minimum essential medium supplemented with 0.1 or 0.2% bovine serum albumin, one hour after the treatment with 0.1 ml solution of the compound. After the incubation for 2 or 3 days at 37°C in 5% $CO_2$, cytopathic effect induced by $100TCID_{50}$ of the virus was observed under a microscope. Minimum inhibitory concentration of the compound was defined as the lowest concentration of the compound which shows complete inhibition against cytopathic effect induced by the virus. The results are shown in Table 2.

Table 2 <u>In Vitro Antiviral Activity</u>

| Compound \ Virus | Minimum Inhibitory Concentration (µg/ml) | | | | | | | | | |
|---|---|---|---|---|---|---|---|---|---|---|
| | A | B | C | D | E | F | G | H | I | J |
| 1 | 30 | 300 | 300 | 300 | 300 | 300 | 100 | 300 | 300 | 300 |
| 2 | 30 | 300 | 300 | 300 | 300 | 300 | 300 | 300 | 100 | 300 |
| 3 | 30 | 300 | 300 | 300 | 300 | 300 | 300 | 300 | 300 | 300 |
| 4 | 30 | 100 | 300 | 300 | 300 | 300 | 300 | 300 | 300 | 300 |
| 5 | 30 | 300 | 300 | 300 | 300 | 300 | 100 | 300 | 300 | 300 |
| 6 | 30 | 300 | 300 | 300 | 300 | 100 | 300 | 300 | 300 | 300 |
| 7 | 10 | 300 | 300 | 100 | 100 | 100 | 100 | 100 | 30 | 100 |

| Note: | | |
|---|---|---|
| | Influenza $A_0$ | A |
| | Influenza $A_1$ | B |
| | Influenza $A_2$ | C |
| | Influenza B | D |
| | Parainfluenza Type 3 | E |
| | ECHO | F |
| | Coxsackie Type $B_5$ | G |
| | Rhino | H |
| | Respiratory virus | I |
| | Vesicular stomatitis | J |

All of the compounds 1 - 7 showed antiviral activity of wide spectrum although each compound had its specificity with respect to viruses on which it is more effective.

Experimental Example 2 Protective Action against the Death of Mice Infected with Influenza $A_0$ Virus.

After the influenza $A_0$ virus was inoculated to groups each consisting of ten ICR female mice (16 - 18 g) intranasally under light anesthesia with ether, compound of the invention was given orally or intraperitoneally as multiple doses of twice a day starting one hour after infection and continued for 14 days. The result of the treatment was shown as the increase in percentage of survivors at 15th day.

The results are shown in Tables 3 and 4.

Table 3 Result of Intraperitoneal Administration

| Compound | Dosage mg/kg | Survival rate % |
|---|---|---|
| Control | | 0 |
| 1 | 100 | 40 |
| 2 | 100 | 40 |
| 3 | 100 | 40 |
| 4 | 100 | 40 |
| 5 | 100 | 40 |
| 6 | 100 | 50 |
| 7 | 10 | 40 |
| | 30 | 50 |

Table 4 Result of Oral Administration

| Compound | Dosage mg/kg | Survival rate % |
|---|---|---|
| Control | | 0 |
| 1 | 300 | 50 |
| 2 | 300 | 50 |
| 3 | 300 | 50 |
| 4 | 300 | 40 |
| 5 | 300 | 40 |
| 6 | 300 | 50 |
| 7 | 30 | 40 |
| | 100 | 60 |

All of the compounds 1 to 7 showed protective action against death due to the infection, and especially, the compound 7 showed extremely excellent activity.

Experimental Example 3: Protective Action against the Death of Mice Infected with Influenza Virus A/WSN

After the influenza virus A/WSN was inoculated to groups each consisting of ten ICR female mice (16 - 18 g) intranasally under light anesthesia with ether, each compound of the invention was given intraperitoneally or orally one hour after the infection. Table 5 shows the results of survival rate observed 15 days after the infection.

All of the compounds examined indicated markedly higher survival rates of the treated groups as compared with that of the control. Efficacy of aminosulfonylbenzoic ester derivatives was supported by these experiments.

Table 5

| Intraperitoneal | | | Oral | | |
|---|---|---|---|---|---|
| Compound | Dosage (mg/Kg) | Survival rate (%) | Compound | Dosage (mg/Kg) | Survival rate (%) |
| Control | | 0 | Control | | 0 |
| Amantadine | 100 | 40 | Amantadine | 300 | 40 |
| 8 | 30<br>100 | 40<br>50 | 9 | 100<br>300 | 30<br>40 |
| 10 | 10<br>30 | 50<br>70 | 10 | 30<br>100 | 50<br>60 |
| 11 | 30<br>100 | 30<br>40 | | | |

Experimental Example 4: Protective Action against the Death of Mice Infected with Influenza Virus B/GL

After the influenza virus B/GL was inoculated to groups each consisting of ten ICR female mice (16 - 18 g) intranasally under light anesthesia with ether, each compound of the invention was given orally one hour after the infection. Table 6 shows the results of survival rate observed 15 days after the infection.

The compounds examined indicated higher survival rates of the treated groups as compared with that of the control. Especially, compound 10 showed extremely excellent activity.

Table 6

| Compound | Dosage mb/kg | Survival rate (%) |
|---|---|---|
| Control | | 0 |
| Amantadine | 300 | 30 |
| 9 | 100 | 30 |
| | 300 | 40 |
| 10 | 30 | 60 |
| | 300 | 70 |

Experimental Example 5: Protective Action against the Death of Mice Infected with Coxsackie Virus $B_1$

After the coxsackie virus $B_1$ was inoculated to groups each consisting of ten ICR female mice (16 - 18 g) intraperitoneally, compound 10 was given orally one hour after the infection. Table 7 shows the results of survival rate observed 10 days after the infection.

Table 7

| Compound | Dosage mg/kg | Survival rate (%) |
|---|---|---|
| Control | | 10 |
| 10 | 300<br>1000 | 40<br>70 |

Experimental Example 6: Protective Action against the Death of Mice Infected with Parainfluenza Virus Type 3

After the parainfluenza virus type 3 was inoculated to groups each consisting of ten ICR female mice (16 - 18 g) intranasally under light anesthesia with ether, compound 10 was given orally one hour after the infection. Table 8 shows the results of survival rate observed 15 days after the infection.

Table 8

| Compound | Dosage mg/kg | Survival rate (%) |
|---|---|---|
| Control | | 0 |
| 10 | 30 | 40 |
| | 100 | 60 |

Experimental Example 7: Acute Toxicity in Mice

Each compound of the invention was suspended in 5% Arabic gum solution and given orally (p.o.) or intraperitoneally (i.p.) to groups of ten ddY male mice weighed from 24 to 26 g at the dosage volume of 10 ml/kg. The mortality rate was observed for 7 days. $LD_{50}$ was determined according to the method of Wilcoxon and Litchfield. The results are shown in Table 9.

Table 9, $LD_{50}$ value (mg/kg)

| Compound | Oral administration | Intra-peritoneal administration |
|---|---|---|
| 1 | >3000 | >1000 |
| 2 | >3000 | >1000 |
| 3 | >3000 | >1000 |
| 4 | >3000 | >1000 |
| 5 | >3000 | 968 |
| 6 | >3000 | >1000 |
| 7 | >3000 | >1000 |
| 8 | >3000 | >1000 |
| 9 | >3000 | >1000 |
| 10 | >3000 | >1000 |
| 11 | >3000 | >1000 |

As apparent from the above experimental examples, all of the compounds 1 - 11 have antiviral activity of wide spectrum and are of extremely high safety. Therefore, they have very high clinical utility for treating various infectious diseases which are caused from virus infection, for example, upper respiratory infection, pneumonia, Brouchitis and so on.

In case that the compounds of the present invention are administerd to a human body, dosage for an adult is 30 - 5,000 mg per day for the compounds 1 - 6 and 8; 10 - 5,000 mg per day for the compounds 7 and 10; 50 - 5,000 mg per day for the compounds 9 and 11.

However, the dosage may be increased or decreased out of the above range according to symptoms, ages, and other conditions.

The compounds 1 - 11 can be formulated as pharmaceutical preparations by conventional method with pharmaceutical carriers, base materials as excipients commonly used for this purpose. Example of solid carriers and excipients usable advantageously herein include common excipients such as lactose, mannitol, corn starch and potato starch, binders such as crystalline cellulose, cellulose derivatives, arabic gum, corn starch and gelatin; disintegrators such as corn starch, potato starch and calcium carbohydroxymethylcellulose; and lubricants such as talc and magnesium stearate. Examples of liquid carriers usable advantageously herein include distilled water for injection, physiological saline solution, vegetable oils for injection and glycols such as propylene glycol and polyethylene glycol.

Such preparations can be for example capsules, tablets, powders or oral liquid preparations (including dry sirups) for oral application; rectum suppository for intrarectal application for injection, they can be for example, freeze-dried preparations which can be dissolved in distilled water for injection immediately before administration; and other preparations such as nose drops or inhalant can also be adopted.

The following are the examples of pharmaceutical preparations, but such preparations should not be limited only to the illustrated ones.

Example 1: Tablets

| | Ingredients | Amounts |
|---|---|---|
| I) | Compound 7 | 50 g |
| II) | Lactose | proper amount |
| III) | Crystalline cellulose | 60 g |
| IV) | Potato starch | 54 g |
| V) | Magnesium stearate | 2 g |
| | | 200 g |

The ingredients (I) - (IV) were homogeneously mixed and 10% paste from the part of the ingredient (IV) which had been previously separated was added to the above mixture to prepare granules, and then the granules were dried. Next, the granules were mixed with the ingredient (V) to provide tablets each weighing 200 mg. If desired, the tablets may be coated with sugar in usual manner.

Example 2: 10% Powders

| Ingredients | Amounts |
|---|---|
| Compound 9 | 100 g |
| Lactose | 890 g |
| Magnesium stearate | 10 g |
| | 1000 g |

After each of the above ingredients was weighed, the ingredients were homogeneously mixed to prepare 5% powders.

Example 3: Capsules

| | Ingredients | Amounts |
|---|---|---|
| I) | Compound 3 | 50 g |
| II) | Calcium hydrogenphosphate | 50 g |
| III) | Aluminum silicate | proper amount |
| IV) | Crystalline cellulose | 60 g |
| V) | Magnesium stearate | 2 g |
| | | 200 g |

The above ingredients (I) - (V) were put together and mixed well through a sieve, to give capsules each weighing 200 mg were prepared from the mixture in usual manner.

Example 4: Injectable solutions

One hundred grams of sodium salt of the Compound 2 were dissolved in 2 ℓ of distilled water for injection and from this solution, injectable solutions which contain 100 mg of the active compound in 2 mℓ of the solution per ampoule were prepared in usual manner.

Example 5: 1% Nose drops

| Ingredients | Amounts |
|---|---|
| Compound 8 (sodium salt) | 10 g |
| Sodium chloride | 5 g |
| Chlorobutanol | 5 g |
| Distilled water added to become | 1,000 mℓ |

After each of the above ingredients was weighed and dissolved together in 950 ml of water, the solution was filled up to become 1,000 ml to prepare 1 % nose drops.

Claims

1. Antiviral composition which contains as an active ingredient an aminosulfonylhalogenobenzoic acid derivative of the general formula (IV)

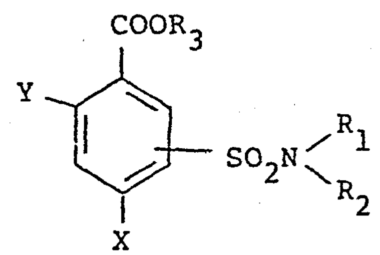

(IV)

wherein X denotes a fluorine atom, a chlorine atom or a bromine atom, Y denotes a hydrogen atom, $R_1$ denotes a hydrogen atom or a methyl group, $R_2$ denotes a hydrogen atom, a methyl group, an ethyl group, an isopropyl group or a phenyl group, or $R_1$ and $R_2$ together with the nitrogen atom in the formula may form a morpholino group, and $R_3$ denotes a hydrogen atom, a methyl group, an isopropyl group or a n-butyl group.

2. Composition according to claim 1, wherein the aminosulfonylhalogenobenzoic acid derivative of the general formula (IV) is a 3-aminosulfonyl-4-chlorobenzoic acid derivative (in the formula, X denotes a chlorine atom, Y denotes a hydrogen atom, $R_1$ denotes a hydrogen atom or a methyl group, $R_2$ denotes a methyl group, an ethyl group, an isopropyl group or a phenyl group, or $R_1$ and $R_2$ together with the nitrogen atom in the formula may form a morpholino group, and $R_3$ denotes a hydrogen atom).

3. Composition according to claim 1, wherein the aminosulfonylhalogenobenzoic acid derivative of the general formula (IV) is a 3-aminosulfonyl-4-halogenobenzoic ester (in the formula, X denotes a fluorine atom, a chlorine atom or a bromine atom, Y denotes a hydrogen atom and both of $R_1$ and $R_2$ denote a hydrogen atom and $R_3$ denotes a methyl group, an ethyl group or an isopropyl group).

4. Antiviral composition which contains as an active ingredient an aminosulfonylhalogenobenzoic acid derivative of the general formula (V):

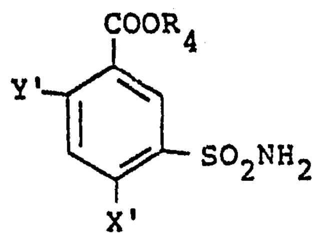

(V)

wherein both of X' and Y' denote a chlorine atom and $R_4$ denotes a hydrogen atom or an allyl group.

5. Composition according to claim 4, wherein the aminosulfonylhalogenobenzoic acid derivative of the general formula (V) is a 5-aminosulfonyl-2,4-dichlorobenzoic acid.

European Patent Office

**0132540**
Application number

# EUROPEAN SEARCH REPORT

EP 84 10 6186

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | GB-A- 915 259 (PARKE & DAVIS)<br>* claims *<br>--- | 1-3 | A 61 K 31/19<br>A 61 K 31/235<br>A 61 K 31/535 |
| X | FR-A-1 311 859 (SIFA)<br>* abstract; page 2, left-hand column *<br>--- | 4,5 | |
| X | CHEMICAL ABSTRACTS, vol. 87, no. 5, 1st August 1977, page 481, no. 39058w, Columbus, Ohio, US; D.A. KULIKOVA et al.: "Relation between the diuretic activity and capacity to prolong the effect of penicillin in a group of some furosemide analogs" & KHIM.-FARM. ZH. 1977, 11(3), 58-61<br>* abstract *<br>--- | 4,5 | TECHNICAL FIELDS SEARCHED (Int. Cl. 3) |
| Y | FR-A-2 106 514 (PFIZER)<br>* claims *<br>--- | 1-5 | A 61 K 31/00<br>C 07 C 143/00 |
| Y | FR-A-2 211 261 (PFIZER)<br>* claims *<br>--- | 1-5 | |
| P,X | FR-A-2 493 702 (MOCHIDA SEIYAKU K.K.)<br>* claims *<br>--- -/- | 1-5 | |

The present search report has been drawn up for all claims

| Place of search | Date of completion of the search | Examiner |
|---|---|---|
| THE HAGUE | 09-10-1984 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

0132540

Application number

European Patent Office

# EUROPEAN SEARCH REPORT

EP 84 10 6186



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| E,X | EP-A-0 068 407 (HODOGAYA CHEMICAL)<br>* claims *<br>----- | 1-5 | |

TECHNICAL FIELDS SEARCHED (Int. Cl. 3)

The present search report has been drawn up for all claims

| Place of search | Date of completion of the search | Examiner |
|---|---|---|
| THE HAGUE | 09-10-1984 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document